# EUROPEAN PATENT APPLICATION

(11) **EP 3 950 605 A1**
(43) Date of publication of application: **09.02.2022**
(21) Application number: 20784308.7
(22) Date of filing: 26.03.2020
(51) Int. Cl.: C02F 1/50, B01F 3/04, B05B 17/04, C09K 3/30, A01N 59/06

(54) **OZONE WATER MANUFACTURING DEVICE, OZONE WATER MANUFACTURING METHOD, OZONE WATER, OZONE WATER PROCESSING DEVICE, AND EVALUATION METHOD**

(30) Priority: 29.03.2019 JP 2019067035; 25.11.2019 JP 2019212054
(71) Applicant: IHI Corporation, Koto-ku Tokyo 135-8710 (JP)
(72) Inventor: KAMASE, Yukihiro, Tokyo 135-8710 (JP); HIRONAKA, Nobuharu, Tokyo 135-8710 (JP)
(74) Representative: O'Connor, Dominic David
(86) International application number: PCT/JP2020/013843
(87) International publication number: WO 2020/203698

(57) **Abstract**

An ozone water production apparatus (100) includes: a solution accommodation unit (110) configured to accommodate a magnesium solution (M) in which 1.0 mass% or more and less than 4.0 mass% of a magnesium compound is dissolved in water; and a bubble introduction unit (120) configured to introduce fine bubbles of ozone into the magnesium solution (M).

## Description

### Technical Field

The present disclosure relates to an ozone water production apparatus, an ozone water production method, ozone water, an ozone water treatment apparatus, and an evaluation method. This application claims the benefit of priority to Japanese Patent Application No. 2019-067035 filed on March 29, 2019 and Japanese Patent Application No. 2019-212054 filed on November 25, 2019, the contents of which are incorporated herein.

### Background Art

Ozone water is used for disinfection, sterilization, deodorization, and the like of objects such as medical equipment. Bubbles of ozone contained in related-art ozone water each have a particle diameter as large as 100 µm or more. For this reason, the period of time during which the related-art ozone water was able to retain ozone in water was as short as from about 1 hour to about 2 hours. Accordingly, it has hitherto been required to subject an object to disinfection, sterilization, deodorization, and the like immediately after the ozone water is produced.

In view of the foregoing, a technology for producing ozone water by generating microbubbles of ozone in water containing 4.0% or more of bittern has been developed (for example, Patent Literature 1).

### Citation List

### Patent Literature

Patent Literature 1: JP 5261569 B2

### Summary

### Technical Problem

However, bittern is produced from a residual liquid after salt is precipitated from seawater, and hence the composition varies depending on the seawater collection location, collection time, and the like. Accordingly, the ozone water as disclosed in Patent Literature 1 produced by supplying ozone to water in which bittern is dissolved has a problem in that the content of the bubbles of ozone is not stable.

In view of the above-mentioned problem, the present disclosure has an object to provide an ozone water production apparatus capable of stabilizing the content of bubbles of ozone while retaining ozone in water for a long period of time, an ozone water production method, ozone water, an ozone water treatment apparatus, and an evaluation method.

### Solution to Problem

In order to solve the above-mentioned problem, according to one aspect of the present disclosure, there is provided an ozone water production apparatus, including: a solution accommodation unit configured to accommodate a magnesium solution in which 1.0 mass% or more and less than 4.0 mass% of a magnesium compound is dissolved in water; and a bubble introduction unit configured to introduce fine bubbles of ozone into the magnesium solution.

In order to solve the above-mentioned problem, according to one aspect of the present disclosure, there is provided an ozone water production method, including introducing fine bubbles of ozone into a magnesium solution in which 1.0 mass% or more and less than 4.0 mass% of a magnesium compound is dissolved in water.

In order to solve the above-mentioned problem, according to one aspect of the present disclosure, there is provided ozone water, including 1.0 mass% or more and less than 4.0 mass% of a magnesium compound and fine bubbles of ozone.

In order to solve the above-mentioned problem, according to one aspect of the present disclosure, there is provided an ozone water treatment apparatus, including a spray unit configured to spray ozone water containing 1.0 mass% or more and less than 4.0 mass% of a magnesium compound and fine bubbles of ozone so as to give a particle diameter of 10 µm or less.

In order to solve the above-mentioned problem, according to one aspect of the present disclosure, there is provided an evaluation method, including the steps of: installing a predetermined number of bacteria at each of a plurality of measurement points having different distances from a spray unit configured to spray ozone water containing 1.0 mass% or more and less than 4.0 mass% of a magnesium compound and fine bubbles of ozone so as to give a particle diameter of 10 µm or less; spraying the ozone water so as to give a particle diameter of 10 µm or less by the spray unit; and counting the number of the bacteria at each of the plurality of measurement points after an elapse of a predetermined period of time from execution of the step of spraying.

### Effects of Disclosure

According to the present disclosure, it is possible to retain ozone in water for a long period of time and to stabilize the content of the bubbles of ozone.

### Brief Description of Drawings

FIG. 1 is a diagram for illustrating an ozone water production apparatus.
FIG. 2 is a flowchart for illustrating a flow of treatment of an ozone water production method.
FIG. 3 is a graph for showing results of a sterilization test using ozone water of Comparative Examples A-1 to A-3 and Comparative Examples B-1 to B-3.
FIG. 4 is a graph for showing results of a sterilization test using ozone water of Examples A-1 to A-4.
FIG. 5 is a graph for showing results of a sterilization test using ozone water of Examples A-5 to A-8.
FIG. 6 is a graph for showing results of a sterilization test using ozone water of Examples B-1 to B-4.
FIG. 7 is a graph for showing results of a sterilization test using ozone water of Examples C-1 to C-4.
FIG. 8 is a graph for showing results of a sterilization test using ozone water of Examples D-1 to D-4.
FIG. 9 is a graph for showing results of a sterilization test using ozone water of Examples E-1 to E-4.
FIG. 10 is a view for illustrating an ozone water treatment apparatus.
FIG. 11 is a flowchart for illustrating a flow of treatment of an evaluation method.
FIG. 12 is a diagram for illustrating an example of a target space and measurement points.
FIG. 13A is a graph showing results of membrane filters to each of which a 10³ cells/ml bacterial solution was added dropwise in the case of using Example F. FIG. 13B is a graph for showing results of membrane filters to each of which a 10⁴ cells/ml bacterial solution was added dropwise in the case of using Example F.
FIG. 14A is a graph for showing results of membrane filters to each of which a 10³ cells/ml bacterial solution was added dropwise in the case of using Comparative Example C. FIG. 14B is a graph for showing results of membrane filters to each of which a 10⁴ cells/ml bacterial solution was added dropwise in the case of using Comparative Example C.
FIG. 15A is a graph for showing results of membrane filters to each of which a 10³ cells/ml bacterial solution was added dropwise in the case of using Comparative Example D. FIG. 15B is a graph for showing results of membrane filters to each of which a 10⁴ cells/ml bacterial solution was added dropwise in the case of using Comparative Example D.
FIG. 16 is a graph for showing bacterial counts in the case of using third Example.

### Description of Embodiments

Now, with reference to the attached drawings, embodiments of the present disclosure are described in detail. Dimensions, materials, and other specific numerical values represented in the embodiments are merely examples used for facilitating the understanding of the disclosure, and do not limit the present disclosure unless otherwise stated. Elements having substantially the same functions and configurations herein and in the drawings are denoted by the same reference symbols to omit redundant description thereof. Further, illustration of elements with no direct relationship to the present disclosure is omitted.

### [Ozone Water Production Apparatus 100]

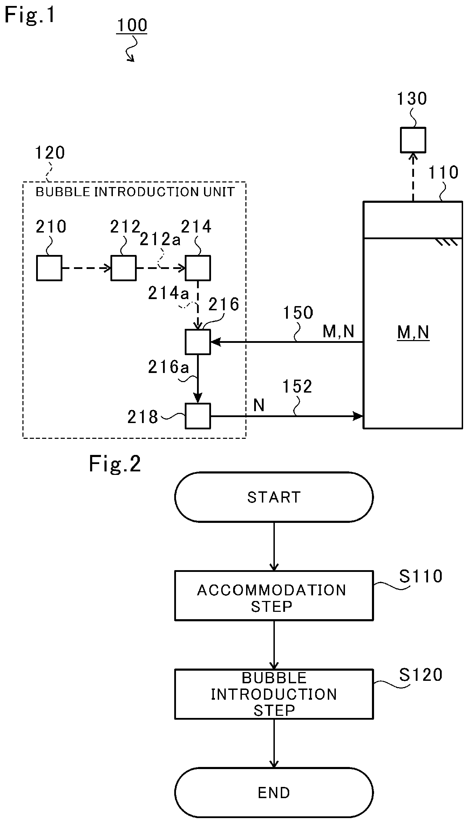

FIG. 1 is a diagram for illustrating an ozone water production apparatus 100. In FIG. 1, the solid arrows each indicate a flow of a magnesium solution M and ozone water N. In FIG. 1, the dashed arrows each indicate a flow of ozone. As illustrated in FIG. 1, the ozone water production apparatus 100 includes a solution accommodation unit 110, a bubble introduction unit 120, and an ozone decomposition unit 130.

The solution accommodation unit 110 is a container configured to accommodate the magnesium solution M. The magnesium solution M is a solution in which 1.0 mass% or more and less than 4.0 mass% of a magnesium compound is dissolved in water. The magnesium compound is, for example, one or a plurality of compounds selected from the group consisting of magnesium chloride (MgCl₂), magnesium oxide (MgO), magnesium bromide (MgBr₂), magnesium nitrate (Mg(NO₃)₂), magnesium sulfate (MgSO₄), and magnesium acetate (Mg(CH₃COO)₂). The water is, for example, tap water, purified water (e.g., filter filtration or activated carbon filtration), or pure water.

The bubble introduction unit 120 is configured to generate fine bubbles of ozone in the magnesium solution M and supply the resultant to the solution accommodation unit 110. The bubble introduction unit 120 is, for example, a swirl flow type device. In this embodiment, the bubble introduction unit 120 includes an ozone generation portion 210, a gas pump 212, a mass flow controller 214, a gas mixing pump 216, and a line mixer 218.

The ozone generation portion 210 is, for example, an ozonizer. When oxygen is exposed to a discharge environment in the ozone generation portion 210, a gas containing ozone (hereinafter simply referred to as "ozone") is generated.

The gas pump 212 is configured to suck and compress (pressure-increase) the ozone generated by the ozone generation portion 210. The gas pump 212 is connected to the mass flow controller 214 through a gas supply pipe 212a. A suction side of the gas pump 212 is connected to the ozone generation portion 210. A discharge side of the gas pump 212 is connected to the mass flow controller 214.

The mass flow controller 214 is configured to control a flow rate of the ozone supplied from the gas pump 212. The mass flow controller 214 is configured to control the flow rate of the ozone to, for example, a predetermined flow rate within a range of 1% or more and 10% or less of a flow rate of the magnesium solution M introduced from an intake pipe 150.

The gas mixing pump 216 is connected to the solution accommodation unit 110 through the intake pipe 150. The gas mixing pump 216 is connected to the mass flow controller 214 through a gas supply pipe 214a. The gas mixing pump 216 is configured to suck the magnesium solution M in the solution accommodation unit 110, and mix the ozone having been supplied from the gas pump 212 and having passed through the mass flow controller 214, with the magnesium solution M.

The line mixer 218 is connected to the gas mixing pump 216 through a water supply pipe 216a. The line mixer 218 is configured to crush the ozone mixed with the magnesium solution M to generate fine bubbles. The fine bubbles are bubbles each having a diameter of 100 µm or less. Of the fine bubbles, bubbles each having a diameter of 1 µm or more and 100 µm or less are referred to as "microbubbles". In addition, of the fine bubbles, bubbles each having a diameter of less than 1 µm are referred to as "ultrafine bubbles".

The magnesium solution M containing the fine bubbles of the ozone, that is, the ozone water N is supplied to the solution accommodation unit 110 through a water supply pipe 152 by the line mixer 218.

Then, the ozone water production apparatus 100 circulates the magnesium solution M and the ozone water N in the solution accommodation unit 110 to the bubble introduction unit 120 (gas mixing pump 216 and line mixer 218). With this configuration, the ozone water production apparatus 100 can produce the ozone water N containing the fine bubbles at a target concentration in the solution accommodation unit 110.

The ozone decomposition unit 130 is connected to an upper portion of the solution accommodation unit 110. The ozone decomposition unit 130 is, for example, a deozonizer. The ozone decomposition unit 130 is configured to decompose the ozone discharged from the solution accommodation unit 110. The gas in which the ozone has been decomposed by the ozone decomposition unit 130 is released into the atmosphere.

### [Ozone Water Production Method]

Subsequently, an ozone water production method using the ozone water production apparatus 100 is described. FIG. 2 is a flowchart for illustrating a flow of treatment of the ozone water production method. As illustrated in FIG. 2, the ozone water production method includes an accommodation step S110 and a bubble introduction step S120.

### [Accommodation Step S110]

The accommodation step S110 is a step of accommodating the magnesium solution M in the solution accommodation unit 110.

### [Bubble Introduction Step S120]

The bubble introduction step S120 is a step of driving the bubble introduction unit 120 to generate the fine bubbles of the ozone in the magnesium solution M. With this configuration, the ozone water N containing 1.0 mass% or more and less than 4.0 mass% of the magnesium compound and the fine bubbles of the ozone is produced.

As described above, in the ozone water production apparatus 100 and the ozone water production method according to this embodiment, the fine bubbles of the ozone are generated in the magnesium solution M in which 1.0 mass% or more and less than 4.0 mass% of the magnesium compound is dissolved in water. When the content of the magnesium compound is less than 1.0 mass%, the content of the fine bubbles of the ozone in the ozone water N becomes low. In addition, even when the content of the magnesium compound is set to 4.0 mass or more, the content of the fine bubbles of the ozone in the ozone water N is hardly increased, but the cost of the magnesium compound itself is increased. Accordingly, in the ozone water production apparatus 100 and the ozone water production method, the ozone water N containing the fine bubbles of the ozone at a high content can be produced at low cost by generating the fine bubbles of the ozone in the magnesium solution M in which 1.0 mass% or more and less than 4.0 mass% of the magnesium compound is dissolved in water.

In addition, unlike bittern, the composition of the magnesium compound is stable. Accordingly, in the ozone water production apparatus 100 and the ozone water production method, the ozone water N containing the fine bubbles of the ozone at a stable content can be produced by generating the fine bubbles of the ozone in the magnesium solution M in which the magnesium compound is dissolved in water. Specifically, in the ozone water production apparatus 100 and the ozone water production method, the ozone water N containing the fine bubbles of the ozone at a target content can be produced.

In addition, when substances other than the magnesium compound are mixed in the solution, it cannot be said that the sterilization performance always deteriorates. However, in the ozone water production apparatus 100 and the ozone water production method, it is preferred that the ozone water N be produced by generating the fine bubbles of the ozone in the magnesium solution M in which only the magnesium compound is dissolved. Specifically, in the ozone water production apparatus 100 and the ozone water production method, for example, the ozone water N containing the fine bubbles of the ozone can be produced even without containing an organic iron compound.

In addition, the ozone water N produced by the ozone water production apparatus 100 and the ozone water production method according to this embodiment contains the fine bubbles of the ozone. The fine bubbles each have a relatively small diameter and hardly float. Specifically, the ozone water N can suppress the release of the fine bubbles of the ozone from water. Thus, the ozone water N can extend the period of time during which the ozone can be retained in water as compared to the related-art ozone water containing ozone having a particle diameter of 100 µm or more. Accordingly, unlike the related-art ozone water, the ozone water N can subject an object to disinfection, sterilization, deodorization, and the like even after the elapse of a certain period of time from the production of the ozone water N.

### [First Example]

Ozone water of each of Comparative Examples A-1 to A-3, Comparative Examples B-1 to B-3, Examples A-1 to A-8, Examples B-1 to B-4, Examples C-1 to C-4, Examples D-1 to D-4, and Examples E-1 to E-4 was prepared. Then, a sterilization test was conducted through use of the above-mentioned ozone water. As the sterilization test, indicator bacteria were loaded into the ozone water and kept at 40°C. As the indicator bacteria, *Bacillus atrophaeus* ATCC 9372 was used. In addition, the number of the indicator bacteria (cfu/ml) was measured before the loading of the indicator bacteria into the ozone water (0 minutes), after the elapse of 20 minutes from the loading, after the elapse of 40 minutes from the loading, and after the elapse of 60 minutes from the loading.

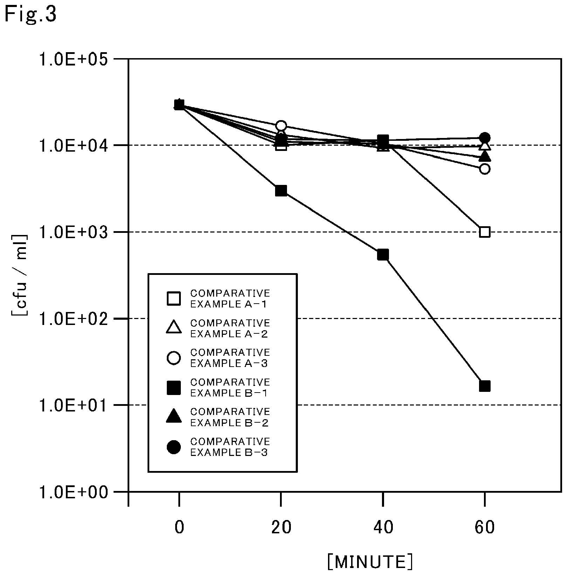

FIG. 3 is a graph for showing results of the sterilization test using the ozone water of each of Comparative Examples A-1 to A-3 and Comparative Examples B-1 to B-3. In FIG. 3, the blank squares indicate Comparative Example A-1. In FIG. 3, the blank triangles indicate Comparative Example A-2. In FIG. 3, the blank circles indicate Comparative Example A-3. In FIG. 3, the solid squares indicate Comparative Example B-1. In FIG. 3, the solid triangles indicate Comparative Example B-2. In FIG. 3, the solid circles indicate Comparative Example B-3.

The ozone water of Comparative Example A-1 is ozone water in which fine bubbles of ozone are generated in water having 4 mass% of bittern dissolved therein. The ozone water of Comparative Example A-2 is obtained by 2-fold dilution of the ozone water of Comparative Example A-1 with water. The ozone water of Comparative Example A-3 is obtained by 5-fold dilution of the ozone water of Comparative Example A-1 with water. The ozone water of Comparative Example B-1 is ozone water in which fine bubbles of ozone are generated in water containing 2 mass% of bittern. The ozone water of Comparative Example B-2 is obtained by 2-fold dilution of the ozone water of Comparative Example B-1 with water. The ozone water of Comparative Example B-3 is obtained by 5-fold dilution of the ozone water of Comparative Example B-1 with water.

In addition, in Comparative Examples A-1 to A-3 and Comparative Examples B-1 to B-3, the sterilization test was conducted on the day on which the ozone water was prepared.

As a result, as shown in FIG. 3, in each of Comparative Examples A-1 to A-3 and Comparative Examples B-1 to B-3, the bacterial count (initial value) at 0 minutes was about 2.9×1.0E04 (10⁴) cfu/ml.

In Comparative Example A-1, the bacterial count after the elapse of 20 minutes was about 1.0×1.0E04 cfu/ml, the bacterial count after the elapse of 40 minutes was about 1.2×1.0E04 cfu/ml, and the bacterial count after the elapse of 60 minutes was about 1.0×1.0E03 (10³) cfu/ml.

In Comparative Example A-2, the bacterial count after the elapse of 20 minutes was about 1.4×1.0E04 cfu/ml, the bacterial count after the elapse of 40 minutes was about 9.3×1.0E03 cfu/ml, and the bacterial count after the elapse of 60 minutes was about 1.0×1.0E04 cfu/ml.

In Comparative Example A-3, the bacterial count after the elapse of 20 minutes was about 1.7×1.0E04 cfu/ml, the bacterial count after the elapse of 40 minutes was about 1.1×1.0E04 cfu/ml, and the bacterial count after the elapse of 60 minutes was about 5.6×1.0E03 cfu/ml.

In Comparative Example B-1, the bacterial count after the elapse of 20 minutes was about 3.1×1.0E03 cfu/ml, the bacterial count after the elapse of 40 minutes was about 5.5×1.0E02 (10²) cfu/ml, and the bacterial count after the elapse of 60 minutes was about 1.7×1.0E01 (10) cfu/ml.

In Comparative Example B-2, the bacterial count after the elapse of 20 minutes was about 1.1×1.0E04 cfu/ml, the bacterial count after the elapse of 40 minutes was about 1.0×1.0E04 cfu/ml, and the bacterial count after the elapse of 60 minutes was about 7.8×1.0E03 cfu/ml.

In Comparative Example B-3, the bacterial count after the elapse of 20 minutes was about 1.2×1.0E04 cfu/ml, the bacterial count after the elapse of 40 minutes was about 1.1×1.0E04 cfu/ml, and the bacterial count after the elapse of 60 minutes was about 1.3×1.0E04 cfu/ml.

As described above, it was recognized that the ozone water of each of the Comparative Examples in which the fine bubbles of the ozone were generated in water having bittern dissolved therein had a low sterilization effect irrespective of the concentration of bittern. In addition, it was found that the ozone water of each of the Comparative Examples had almost no sterilization effect when diluted with water irrespective of the concentration of bittern.

In addition, there was obtained the result that the ozone water containing bittern at a concentration of 2 mass% had a sterilization effect higher than that of the ozone water containing bittern at a concentration of 4 mass%. It is presumed that the composition of bittern is not constant, and hence the content of the ozone in the ozone water cannot be increased even when the concentration of bittern is increased.

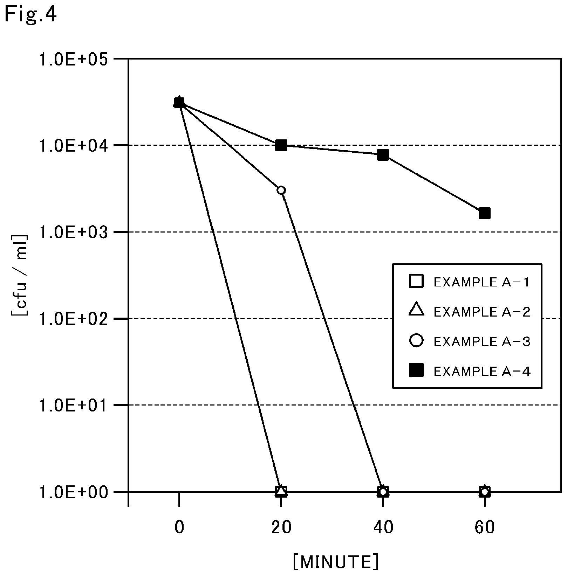

FIG. 4 is a graph for showing results of the sterilization test using the ozone water of each of Examples A-1 to A-4. In FIG. 4, the blank squares indicate Example A-1. In FIG. 4, the blank triangles indicate Example A-2. In FIG. 4, the blank circles indicate Example A-3. In FIG. 4, the solid squares indicate Example A-4.

The ozone water of Example A-1 is ozone water in which fine bubbles of ozone are generated in water having 1 mass% (10 g/l) of magnesium chloride, 1 mass% (10 g/l) of sodium chloride (NaCl), and 0.4 mass% (4 g/l) of potassium chloride (KCl) dissolved therein. The ozone water of Example A-2 is obtained by 2-fold dilution of the ozone water of Example A-1 with water. The ozone water of Example A-3 is obtained by 5-fold dilution of the ozone water of Example A-1 with water. The ozone water of Example A-4 is obtained by 10-fold dilution of the ozone water of Example A-1 with water.

In addition, in Examples A-1 to A-4, the sterilization test was conducted 6 days after the day on which the ozone water was prepared.

As a result, as shown in FIG. 4, in each of Examples A-1 to A-4, the bacterial count (initial value) at 0 minutes was about 3.0×1.0E04 cfu/ml.

In Examples A-1 and A-2, the presence of bacteria was not recognized at any time after the elapse of 20 minutes, after the elapse of 40 minutes, and after the elapse of 60 minutes.

In Example A-3, the bacterial count after the elapse of 20 minutes was about 3.0×1.0E03 cfu/ml, and the presence of bacteria was not recognized after the elapse of 40 minutes and after the elapse of 60 minutes.

In Example A-4, the bacterial count after the elapse of 20 minutes was about 9.7×1.0E03 cfu/ml, the bacterial count after the elapse of 40 minutes was about 7.9×1.0E03 cfu/ml, and the bacterial count after the elapse of 60 minutes was about 1.6×1.0E03 cfu/ml.

As described above, it was recognized that the ozone water of Example A-1 in which the fine bubbles of the ozone were generated in water having 1 mass% of magnesium chloride, 1 mass% of sodium chloride, and 0.4 mass% of potassium chloride dissolved therein was able to perform sterilization in 20 minutes. In addition, it was found that even the ozone water (Example A-2) obtained by 2-fold dilution of the ozone water of Example A-1 was able to perform sterilization in 20 minutes as with the undiluted ozone water (Example A-1).

Further, it was recognized that even the ozone water (Example A-3) obtained by 5-fold dilution of the ozone water of Example A-1 was able to perform sterilization in 40 minutes. In addition, it was found that, in the ozone water (Example A-4) obtained by 10-fold dilution of the ozone water of Example A-1, the sterilization effect was improved by extending the treatment time.

When the dilution rate with water is increased, the sterilization effect is decreased. Accordingly, it is presumed that the content of the ozone is decreased due to the dilution.

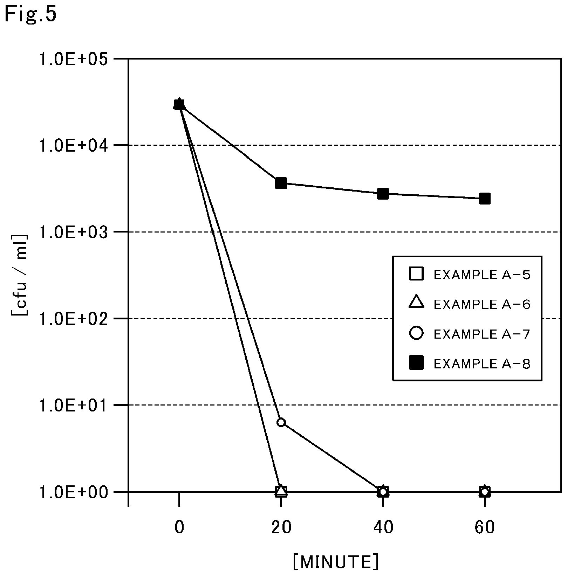

FIG. 5 is a graph for showing results of the sterilization test using the ozone water of each of Examples A-5 to A-8. In FIG. 5, the blank squares indicate Example A-5. In FIG. 5, the blank triangles indicate Example A-6. In FIG. 5, the blank circles indicate Example A-7. In FIG. 5, the solid squares indicate Example A-8.

The ozone water of Example A-5 is the same as that of Example A-1. The ozone water of Example A-6 is the same as that of Example A-2. The ozone water of Example A-7 is the same as that of Example A-3. The ozone water of Example A-8 is the same as that of Example A-4. Unlike in Examples A1 to A-4, in Examples A-5 to A-8, the sterilization test was conducted 12 days after the day on which the ozone water was prepared.

As a result, as shown in FIG. 5, in each of Examples A-5 to A-8, the bacterial count (initial value) at 0 minutes was about 3.0×1.0E04 cfu/ml.

In Examples A-5 and A-6, the presence of bacteria was not recognized at any time after the elapse of 20 minutes, after the elapse of 40 minutes, and after the elapse of 60 minutes.

In Example A-7, the bacterial count after the elapse of 20 minutes was about 6.7 cfu/ml, and the presence of bacteria was not recognized after the elapse of 40 minutes and after the elapse of 60 minutes.

In Example A-8, the bacterial count after the elapse of 20 minutes was about 3.7×1.0E03 cfu/ml, the bacterial count after the elapse of 40 minutes was about 2.9×1.0E03 cfu/ml, and the bacterial count after the elapse of 60 minutes was about 2.4×1.0E03 cfu/ml.

As described above, it was recognized that the ozone water of Example A-5 in which the fine bubbles of the ozone were generated in water having 1 mass% of magnesium chloride, 1 mass% of sodium chloride, and 0.4 mass% of potassium chloride dissolved therein had a sterilization effect substantially equal to that of Example A-1 even after the lapse of time from the generation of the ozone water.

In addition, it was found that the ozone water (Example A-6) obtained by 2-fold dilution of the ozone water of Example A-5 had a sterilization effect substantially equal to that of Example A-2 even after the lapse of time from the generation of the ozone water.

Similarly, it was recognized that each of the ozone water (Example A-7) obtained by 5-fold dilution of the ozone water of Example A-5 and the ozone water (Example A-8) obtained by 10-fold dilution of the ozone water of Example A-5 had a sterilization effect substantially equal to those of Examples A-3 and A-4 even after the lapse of time from the generation of the ozone water.

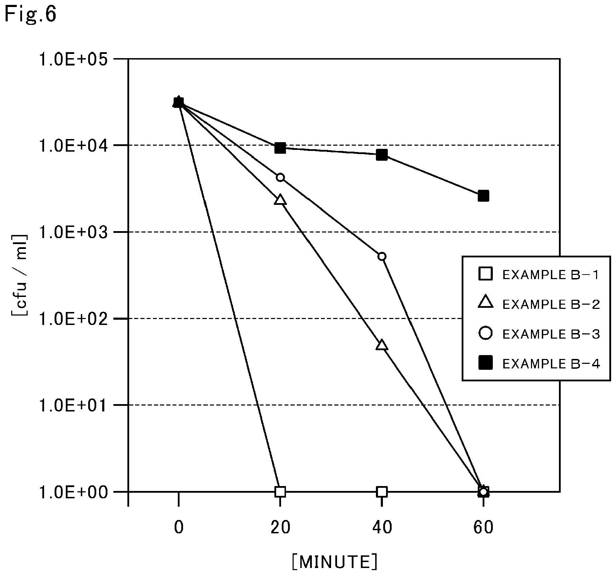

FIG. 6 is a graph for showing results of the sterilization test using the ozone water of each of Examples B-1 to B-4. In FIG. 6, the blank squares indicate Example B-1. In FIG. 6, the blank triangles indicate Example B-2. In FIG. 6, the blank circles indicate Example B-3. In FIG. 6, the solid squares indicate Example B-4.

The ozone water of Example B-1 is ozone water in which fine bubbles of ozone are generated in water having 1 mass% (10 g/l) of magnesium chloride dissolved therein. The ozone water of Example B-2 is obtained by 2-fold dilution of the ozone water of Example B-1 with water. The ozone water of Example B-3 is obtained by 5-fold dilution of the ozone water of Example B-1 with water. The ozone water of Example B-4 is obtained by 10-fold dilution of the ozone water of Example B-1 with water.

In addition, in Examples B-1 to B-4, the sterilization test was conducted 6 days after the day on which the ozone water was prepared.

As a result, as shown in FIG. 6, in each of Examples B-1 to B-4, the bacterial count (initial value) at 0 minutes was about 3.0×1.0E04 cfu/ml.

In Example B-1, the presence of bacteria was not recognized at any time after the elapse of 20 minutes, after the elapse of 40 minutes, and after the elapse of 60 minutes.

In Example B-2, the bacterial count after the elapse of 20 minutes was about 2.4×1.0E03 cfu/ml, the bacterial count after the elapse of 40 minutes was about 5.0×10 cfu/ml, and the presence of bacteria was not recognized after the elapse of 60 minutes.

In Example B-3, the bacterial count after the elapse of 20 minutes was about 4.3×1.0E03 cfu/ml, the bacterial count after the elapse of 40 minutes was about 5.3×1.0E02 cfu/ml, and the presence of bacteria was not recognized after the elapse of 60 minutes.

In Example B-4, the bacterial count after the elapse of 20 minutes was about 9.6×1.0E03 cfu/ml, the bacterial count after the elapse of 40 minutes was about 8.4×1.0E03 cfu/ml, and the bacterial count after the elapse of 60 minutes was about 2.5×1.0E03 cfu/ml.

As described above, it was recognized that the ozone water of Example B-1 in which the fine bubbles of the ozone were generated in water having 1 mass% of magnesium chloride dissolved therein was able to perform sterilization in 20 minutes. In addition, it was found that even the ozone water (Example B-2) obtained by 2-fold dilution of the ozone water of Example B-1 and the ozone water (Example B-3) obtained by 5-fold dilution of the ozone water of Example B-1 were able to perform sterilization in 60 minutes.

Further, it was found that, in the ozone water (Example B-4) obtained by 10-fold dilution of the ozone water of Example B-1, the sterilization effect was improved by extending the treatment time.

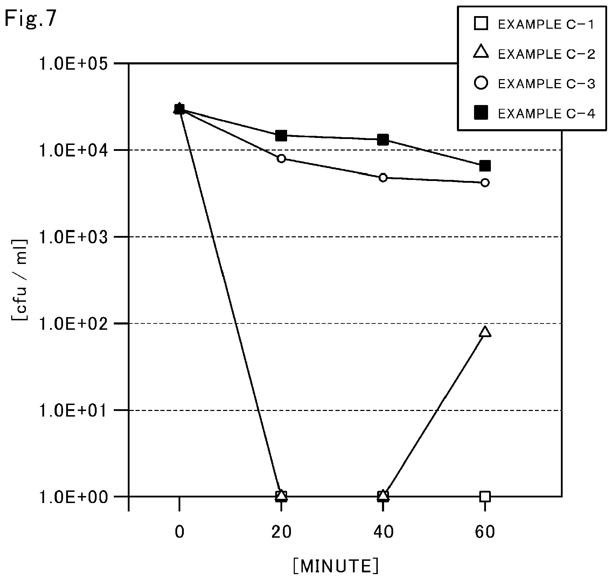

FIG. 7 is a graph for showing results of the sterilization test using the ozone water of each of Examples C-1 to C-4. In FIG. 7, the blank squares indicate Example C-1. In FIG. 7, the blank triangles indicate Example C-2. In FIG. 7, the blank circles indicate Example C-3. In FIG. 7, the solid squares indicate Example C-4.

The ozone water of Example C-1 is ozone water in which fine bubbles of ozone are generated in water having 1 mass% (10 g/l) of sodium chloride dissolved therein. The ozone water of Example C-2 is obtained by 2-fold dilution of the ozone water of Example C-1 with water. The ozone water of Example C-3 is obtained by 5-fold dilution of the ozone water of Example C-1 with water. The ozone water of Example C-4 is obtained by 10-fold dilution of the ozone water of Example C-1 with water.

In addition, in Examples C-1 to C-4, the sterilization test was conducted 7 days after the day on which the ozone water was prepared.

As a result, as shown in FIG. 7, in each of Examples C-1 to C-4, the bacterial count (initial value) at 0 minutes was about 3.0×1.0E04 cfu/ml.

In Example C-1, the presence of bacteria was not recognized at any time after the elapse of 20 minutes, after the elapse of 40 minutes, and after the elapse of 60 minutes.

In Example C-2, the presence of bacteria was not recognized at any time after the elapse of 20 minutes and after the elapse of 40 minutes.

In Example C-3, the bacterial count after the elapse of 20 minutes was about 8.7×1.0E03 cfu/ml, the bacterial count after the elapse of 40 minutes was about 4.9×1.0E03 cfu/ml, and the bacterial count after the elapse of 60 minutes was about 4.2×1.0E03 cfu/ml.

In Example C-4, the bacterial count after the elapse of 20 minutes was about 1.5×1.0E04 cfu/ml, the bacterial count after the elapse of 40 minutes was about 1.3×1.0E04 cfu/ml, and the bacterial count after the elapse of 60 minutes was about 6.6×1.0E03 cfu/ml.

As described above, it was recognized that the ozone water of Example C-1 in which the fine bubbles of the ozone were generated in water having 1 mass% of sodium chloride dissolved therein was able to perform sterilization in 20 minutes. In addition, it was found that even the ozone water (Example C-2) obtained by 2-fold dilution of the ozone water of Example C-1 was able to perform sterilization in 20 minutes as with the undiluted ozone water (Example C-1).

In addition, it was found that, in the ozone water (Example C-3) obtained by 5-fold dilution of the ozone water of Example C-1 and the ozone water (Example C-4) obtained by 10-fold dilution of the ozone water of Example C-1, the sterilization effect was decreased as compared to those of Examples B-3 and B-4, but the sterilization effect was improved by extending the treatment time.

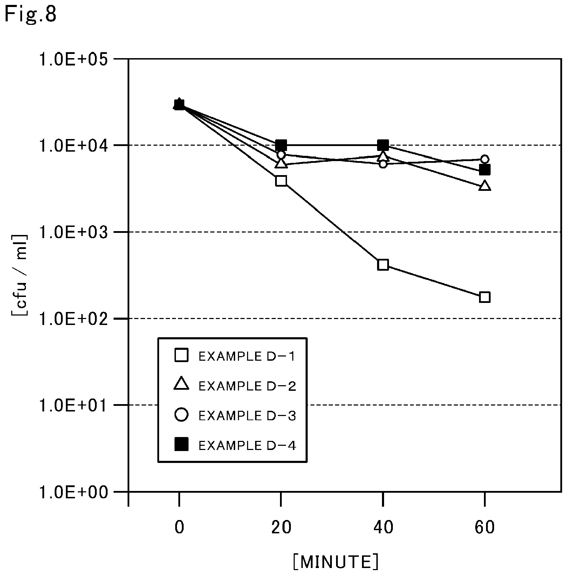

FIG. 8 is a graph for showing results of the sterilization test using the ozone water of each of Examples D-1 to D-4. In FIG. 8, the blank squares indicate Example D-1. In FIG. 8, the blank triangles indicate Example D-2. In FIG. 8, the blank circles indicate Example D-3. In FIG. 8, the solid squares indicate Example D-4.

The ozone water of Example D-1 is ozone water in which fine bubbles of ozone are generated in water having 0.4 mass% (4 g/l) of potassium chloride dissolved therein. The ozone water of Example D-2 is obtained by 2-fold dilution of the ozone water of Example D-1 with water. The ozone water of Example D-3 is obtained by 5-fold dilution of the ozone water of Example D-1 with water. The ozone water of Example D-4 is obtained by 10-fold dilution of the ozone water of Example D-1 with water.

In addition, in Examples D-1 to D-4, the sterilization test was conducted 7 days after the day on which the ozone water was prepared.

As a result, as shown in FIG. 8, in each of Examples D-1 to D-4, the bacterial count (initial value) at 0 minutes was about 3.0×1.0E04 cfu/ml.

In Example D-1, the bacterial count after the elapse of 20 minutes was about 3.9×1.0E03 cfu/ml, the bacterial count after the elapse of 40 minutes was about 4.1×1.0E02 cfu/ml, and the bacterial count after the elapse of 60 minutes was about 1.8×1.0E02 cfu/ml.

In Example D-2, the bacterial count after the elapse of 20 minutes was about 6.4×1.0E03 cfu/ml, the bacterial count after the elapse of 40 minutes was about 7.5×1.0E03 cfu/ml, and the bacterial count after the elapse of 60 minutes was about 3.4×1.0E03 cfu/ml.

In Example D-3, the bacterial count after the elapse of 20 minutes was about 7.6×1.0E03 cfu/ml, the bacterial count after the elapse of 40 minutes was about 6.0×1.0E03 cfu/ml, and the bacterial count after the elapse of 60 minutes was about 6.9×1.0E03 cfu/ml.

In Example D-4, the bacterial count after the elapse of 20 minutes was about 1.0×1.0E04 cfu/ml, the bacterial count after the elapse of 40 minutes was about 9.7×1.0E03 cfu/ml, and the bacterial count after the elapse of 60 minutes was about 5.4×1.0E03 cfu/ml.

As described above, it was recognized that, in the ozone water of each of Examples D-1 to D-4 in which the fine bubbles of the ozone were generated in water having 0.4 mass% of potassium chloride dissolved therein, the sterilization effect was decreased as compared to those of Examples B-1 to B-4 and Examples C-1 to C-4, but the sterilization effect was improved by extending the treatment time.

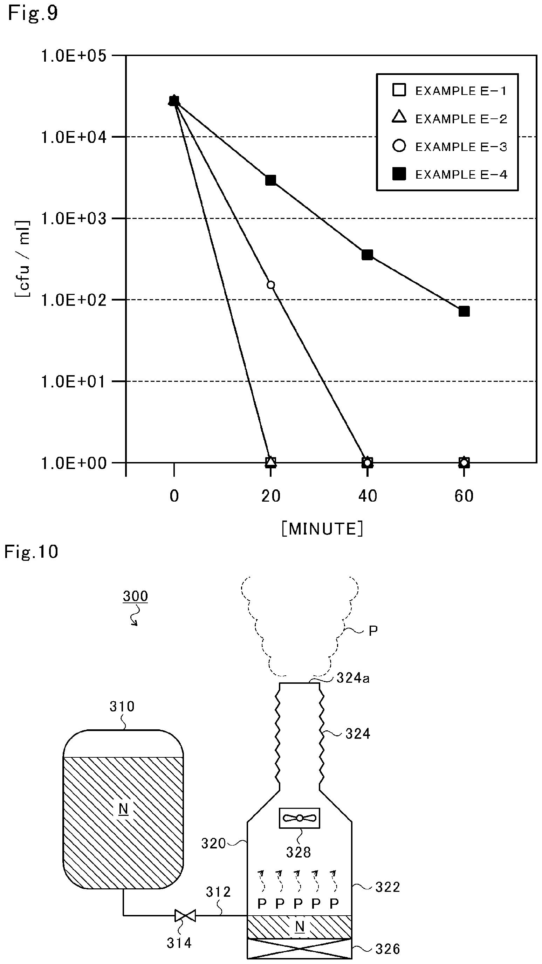

FIG. 9 is a graph for showing results of the sterilization test using the ozone water of each of Examples E-1 to E-4. In FIG. 9, the blank squares indicate Example E-1. In FIG. 9, the blank triangles indicate Example E-2. In FIG. 9, the blank circles indicate Example E-3. In FIG. 9, the solid squares indicate Example E-4.

The ozone water of Example E-1 is ozone water in which fine bubbles of ozone are generated in water having 2 mass% (20 g/l) of magnesium chloride, 1 mass% (10 g/l) of sodium chloride (NaCl), and 0.4 mass% (4 g/l) of potassium chloride (KCl) dissolved therein. The ozone water of Example E-2 is obtained by 2-fold dilution of the ozone water of Example E-1 with water. The ozone water of Example E-3 is obtained by 5-fold dilution of the ozone water of Example E-1 with water. The ozone water of Example E-4 is obtained by 10-fold dilution of the ozone water of Example E-1 with water.

In addition, in Examples E-1 to E-4, the sterilization test was conducted 1 day after the day on which the ozone water was prepared.

As a result, as shown in FIG. 9, in each of Examples E-1 to E-4, the bacterial count (initial value) at 0 minutes was about 3.0×1.0E04 cfu/ml.

In Examples E-1 and E-2, the presence of bacteria was not recognized at any time after the elapse of 20 minutes, after the elapse of 40 minutes, and after the elapse of 60 minutes.

In Example E-3, the bacterial count after the elapse of 20 minutes was about 1.6×1.0E02 cfu/ml, and the presence of bacteria was not recognized after the elapse of 40 minutes and after the elapse of 60 minutes.

In Example E-4, the bacterial count after the elapse of 20 minutes was about 3.0×1.0E03 cfu/ml, the bacterial count after the elapse of 40 minutes was about 3.7×1.0E02 cfu/ml, and the bacterial count after the elapse of 60 minutes was about 7.3×10 cfu/ml.

As described above, it was recognized that the ozone water of Example E-1 in which the fine bubbles of the ozone were generated in water having 2 mass% of magnesium chloride, 1 mass% of sodium chloride, and 0.4 mass% of potassium chloride dissolved therein was able to perform sterilization in 20 minutes. In addition, it was found that even the ozone water (Example E-2) obtained by 2-fold dilution of the ozone water of Example E-1 was able to perform sterilization in 20 minutes as with the undiluted ozone water (Example E-1).

Further, it was recognized that even the ozone water (Example E-3) obtained by 5-fold dilution of the ozone water of Example E-1 was able to perform sterilization in 40 minutes. In addition, it was found that, in the ozone water (Example E-4) obtained by 10-fold dilution of the ozone water of Example E-1, the sterilization effect was improved by extending the treatment time.

In addition, it was recognized that each of Examples E-1 to E-4 had a sterilization effect higher than those of Examples A-1 to A-4. From the above-mentioned results, it was found that ozone water containing fine bubbles of ozone at a high content was able to be produced by supplying ozone to water containing a magnesium compound at a high concentration.

### [Ozone Water Treatment Apparatus]

FIG. 10 is a view for illustrating an ozone water treatment apparatus 300. In FIG. 10, hatching indicates the ozone water N. As illustrated in FIG. 10, the ozone water treatment apparatus 300 includes an ozone water tank 310, a connection pipe 312, an on-off valve 314, and a spray unit 320.

The ozone water tank 310 is configured to store the ozone water N. As described above, the ozone water N contains 1.0 mass% or more and less than 4.0 mass% of a magnesium compound and fine bubbles of ozone.

The connection pipe 312 is configured to connect the ozone water tank 310 and a main body 322 of the spray unit 320 to each other. The on-off valve 314 is provided to the connection pipe 312. The on-off valve 314 is opened by an on-off valve control mechanism (not shown) when the water level (water amount) of the ozone water N stored in the main body 322 is less than a predetermined value. In addition, the on-off valve 314 is closed by the on-off valve control mechanism when the water level of the ozone water N stored in the main body 322 is equal to or more than the predetermined value.

The spray unit 320 is configured to spray ozone water so as to give a particle diameter of 10 µm or less, preferably 3 µm or more and 5 µm or less. In this embodiment, the spray unit 320 includes the main body 322, a particle diameter adjusting mechanism 324, a heating portion 326, and an air blower portion 328.

The main body 322 is a container configured to store the ozone water N. The main body 322 has, for example, a cylindrical shape.

The particle diameter adjusting mechanism 324 is continuous with an upper portion of the main body 322. The particle diameter adjusting mechanism 324 is a cylindrical tube. The particle diameter adjusting mechanism 324 has a diameter smaller than that of the main body 322. The particle diameter adjusting mechanism 324 is expandable and contractible. The particle diameter adjusting mechanism 324 is, for example, a tube formed of bellows (cornice).

The heating portion 326 is, for example, an electric heater. The heating portion 326 is configured to heat the ozone water N accommodated in the main body 322. With this configuration, the ozone water N is atomized into particles P each having a particle diameter of 10 µm or less.

The air blower portion 328 is provided in the main body 322. The air blower portion 328 is provided above the ozone water N stored in the main body 322. The air blower portion 328 is configured to guide the particles P of the ozone water N atomized by the heating portion 326 to the particle diameter adjusting mechanism 324.

The rising distances (moving distances) of the particles P of the ozone water N guided to the particle diameter adjusting mechanism 324 by the air blower portion 328 vary depending on the particle diameters. Specifically, when the particle diameter is larger, the rising distance of the particles P is shorter. When the particle diameter is smaller, the rising distance of the particles P is longer. Accordingly, the length of the particle diameter adjusting mechanism 324 in this embodiment is adjusted so that the particles P each having a particle diameter of 10 µm reach at least an opening 324a of the particle diameter adjusting mechanism 324, and the particles P each having a particle diameter of more than 10 µm do not reach the opening 324a. With this configuration, the ozone water treatment apparatus 300 can send the particles P each having a particle diameter of 10 µm or less to the outside.

As described above, the ozone water treatment apparatus 300 according to this embodiment can supply the particles P of the ozone water N each having a particle diameter of 10 µm or less. Accordingly, the ozone water treatment apparatus 300 can subject a target space, such as a room in which the ozone water treatment apparatus 300 is installed, to disinfection, sterilization, deodorization, bleaching, and the like.

The related-art spray apparatus was configured to spray ozone water having a particle diameter of more than 10 µm. Accordingly, the range in which the related-art spray apparatus can spray ozone water was narrow. In addition, the ozone water sprayed by the related-art spray apparatus has a large particle diameter, and hence the floor around the spray apparatus is wetted with the ozone water. Then, there is a problem in that the floor is discolored or the floor is damaged with the ozone water.

In contrast, the ozone water treatment apparatus 300 according to this embodiment is configured to spray the particles P of the ozone water N each having a particle diameter of 10 µm or less. Accordingly, in the ozone water treatment apparatus 300, the range in which the ozone water N can be sprayed (diffused) can be enlarged as compared to the related-art spray apparatus.

In addition, in the ozone water treatment apparatus 300, the particle diameter of the particles P to be sprayed is smaller than that of the related-art spray apparatus. Accordingly, unlike the related-art spray apparatus, the ozone water treatment apparatus 300 can avoid the situation in which a large amount of the particles P fall to the vicinity of the ozone water treatment apparatus 300. With this configuration, unlike the related-art spray apparatus, the ozone water treatment apparatus 300 can avoid the situation in which the floor around the ozone water treatment apparatus 300 is wetted with the ozone water N. Accordingly, the ozone water treatment apparatus 300 can prevent discoloration of the floor around the ozone water treatment apparatus 300 and damage to the floor.

Further, the particles P sprayed by the ozone water treatment apparatus 300 according to this embodiment each have a large surface area per unit volume as compared to the particles sprayed by the related-art spray apparatus. The effects such as sterilization and deodorization are proportional to the surface areas of the particles of the ozone water. Accordingly, the ozone water treatment apparatus 300 can efficiently perform sterilization, deodorization, and the like as compared to the related-art spray apparatus. Accordingly, the ozone water treatment apparatus 300 can subject a target space to sterilization, deodorization, and the like at low cost as compared to the related-art spray apparatus.

In addition, the related art involving subjecting a target space to sterilization, deodorization, and the like through use of an ozonizer configured to generate an ozone gas has a limit on the concentration of an ozone gas that can be generated. Accordingly, the related art involving using an ozonizer had a problem in that a plurality of ozonizers may be installed or the treatment time by the ozonizer may be prolonged depending on the size of a target space, the amount of bacteria and the degree of odor in the target space, and the like.

In contrast, the ozone water N can retain ozone at a concentration higher than that of an ozone gas generated by an ozonizer for a long time (long period of time). Thus, the ozone water treatment apparatus 300 according to this embodiment can adjust the concentration of ozone supplied to the target space only by adjusting the concentration of the fine bubbles of the ozone contained in the ozone water N to be atomized. Accordingly, the number of the ozone water treatment apparatuses 300 to be installed can be reduced as compared to the related art involving using an ozonizer. In addition, the ozone water treatment apparatus 300 can shorten the treatment time (spray time of the particles P) in the target space as compared to the related art involving using an ozonizer.

In addition, as described above, the spray unit 320 includes the heating portion 326. With this configuration, the ozone water treatment apparatus 300 can generate the particles P at low cost. In addition, the ozone water treatment apparatus 300 can downsize the spray unit 320.

### [Evaluation Method]

An efficient procedure for evaluating the effects such as sterilization and deodorization when particles of ozone water are sprayed to a target space has not hitherto been established. In view of the foregoing, in this embodiment, an evaluation method capable of efficiently evaluating the effects such as sterilization and deodorization in the target space is described.

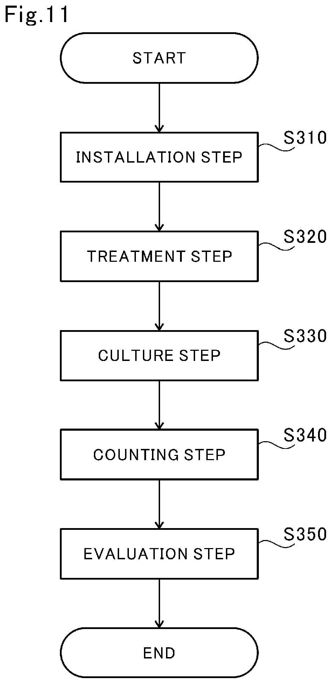

FIG. 11 is a flowchart for illustrating a flow of treatment of the evaluation method according to this embodiment. As illustrated in FIG. 11, the evaluation method includes an installation step S310, a treatment step S320, a culture step S330, a counting step S340, and an evaluation step S350.

### [Installation Step S310]

The installation step S310 is a step of installing a predetermined number of bacteria at each of a plurality of measurement points having different distances from the ozone water treatment apparatus 300 (opening 324a of the spray unit 320) .

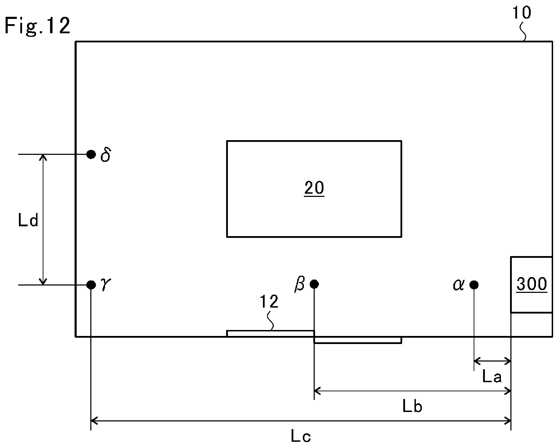

FIG. 12 is a diagram for illustrating an example of a target space 10 and measurement points α to δ. As illustrated in FIG. 12, the target space 10 is, for example, a room provided with a door 12. An installation object 20 (for example, a table) may be provided in the target space 10. The ozone water treatment apparatus 300 is installed in the target space 10.

The plurality of measurement points α to δ have different distances from the ozone water treatment apparatus 300. For example, the measurement point α, the measurement point β, and the measurement point γ are arranged on the same straight line. A distance La between the ozone water treatment apparatus 300 and the measurement point α is shorter than a distance Lb between the ozone water treatment apparatus 300 and the measurement point β. The distance Lb between the ozone water treatment apparatus 300 and the measurement point β is shorter than a distance Lc between the ozone water treatment apparatus 300 and the measurement point γ.

In addition, the measurement point δ is arranged on a line intersecting with (being orthogonal to), on the measurement point γ, the straight line on which the measurement point α, the measurement point β, and the measurement point γ are arranged. The measurement point γ and the measurement point δ are separated by a distance Ld.

Moreover, in the installation step S310, a membrane filter holding a predetermined number of bacteria is installed at each of the measurement points α to δ.

### [Treatment Step S320]

The treatment step S320 is a step of starting the drive of the heating portion 326 of the spray unit 320 to spray the ozone water N so as to give a particle diameter of 10 µm or less, to thereby diffuse the particles P into the target space 10.

### [Culture Step S330]

The culture step S330 is a step of taking out the membrane filters installed at the measurement points α to δ from the target space 10 after the lapse of a predetermined period of time from the start of the treatment step S320 and culturing the bacteria together with the membrane filters. The predetermined period of time is, for example, a predetermined period of time of 30 minutes or more and 60 minutes or less.

In addition, in the culture step S330, a membrane filter holding a predetermined number of bacteria, which has not been subjected to the treatment step S320, is cultured as a positive control.

### [Counting Step S340]

The counting step S340 is a step of counting the number of bacteria (bacterial count) held in each of the membrane filters cultured in the culture step S330.

### [Evaluation Step S350]

The evaluation step S350 is a step of evaluating the effects such as sterilization and deodorization based on the bacterial count counted in the counting step S340. For example, in the evaluation step S350, the bacterial count in the positive control is compared to that in each of the membrane filters installed at the measurement points α to δ. Then, when the difference between the bacterial count in the positive control and the bacterial count in each of the membrane filters installed at the measurement points α to δ is equal to or more than a predetermined value, it is determined that there are effects such as sterilization and deodorization. Meanwhile, when the difference between the bacterial count in the positive control and the bacterial count in each of the membrane filters installed at the measurement points α to δ is less than the predetermined value, it is determined that there are no effects such as sterilization and deodorization.

As described above, in the evaluation method according to this embodiment, a predetermined number of bacteria are installed at each of a plurality of measurement points having different distances from the ozone water treatment apparatus 300 (spray unit 320), and the ozone water treatment apparatus 300 is driven. Then, in the evaluation method, the effects such as sterilization and deodorization are evaluated based on an increase or decrease in number of bacteria at each of the measurement points after the lapse of a predetermined period of time from the start of drive of the ozone water treatment apparatus 300. With this configuration, the evaluation method enables the quantitative evaluation of the sterilization effect of the particles P of the ozone water N at each of the measurement points. Specifically, the evaluation method enables the quantitative evaluation of the effect of deodorizing odor derived from bacteria of the particles P of the ozone water N.

In addition, in the evaluation method according to this embodiment, a predetermined number of bacteria are installed at each of a plurality of measurement points having different distances from the ozone water treatment apparatus 300. With this configuration, the evaluation method enables the evaluation of the effects such as sterilization and deodorization at each of the measurement points of the particles P of the ozone water N. In addition, the evaluation method enables the estimation of the effects such as sterilization and deodorization at a location where the distance from the ozone water treatment apparatus 300 is the same as a predetermined measurement point.

### [Second Example]

The sterilization effect of each of Example F, Comparative Example C, and Comparative Example D by the ozone water treatment apparatus 300 was evaluated through use of the above-mentioned evaluation method.

1.0 ml of a 10³ cells/ml bacterial solution was added dropwise to a membrane filter, and 1.0 ml of a 10⁴ cells/ml bacterial solution was added dropwise to a membrane filter. Each of the membrane filters was accommodated in a perforated petri dish and installed at each of the measurement points α to δ of the target space 10. The distance La between the ozone water treatment apparatus 300 and the measurement point α was set to 10 cm. The distance Lb between the ozone water treatment apparatus 300 and the measurement point β was set to 1.5 m. The distance Lc between the ozone water treatment apparatus 300 and the measurement point γ was set to 3 m. The distance Ld between the measurement point γ and the measurement point δ was set to 1 m. As the membrane filter, a membrane filter having a pore size of 0.45 µm and a diameter of 47 mm was used.

Moreover, ozone water of each of Example F, Comparative Example C, and Comparative Example D was supplied to the ozone water treatment apparatus 300. After the temperature in the target space 10 was stabilized at a predetermined temperature of 20°C or more, and the relative humidity therein was stabilized at a predetermined relative humidity of 65% or more, the drive of the heating portion 326 was started.

The ozone water of Example F is ozone water in which fine bubbles of ozone are generated in water having 1 mass% (10 g/l) of magnesium chloride dissolved therein. The ozone water of Comparative Example C is ozone water in which ultrafine ozone bubbles of ozone are generated in water having 3.25 g/l of sodium (equivalent to 8.3 g/l of salt) dissolved therein. The ozone water of Comparative Example D is ozone water in which nanobubbles of ozone are generated in water having 5.1 g/l of sodium dissolved therein. The number of bubbles in Example F was 160 million pieces/ml. The concentration of the ozone in Example F was 3 mg/l. The number of bubbles in Comparative Example C was 20 million pieces/ml. The concentration of the ozone in Comparative Example C was 2 mg/l. The number of bubbles in Comparative Example D was 120 million pieces/ml. The concentration of the ozone in Comparative Example D was 3 mg/l.

Then, the membrane filter was taken out 0 minutes, 30 minutes, and 45 minutes after the drive of the heating portion 326 was started (treatment time). The membrane filter having been taken out was cultured on an agar medium. Then, the bacterial count on the cultured membrane filter was counted.

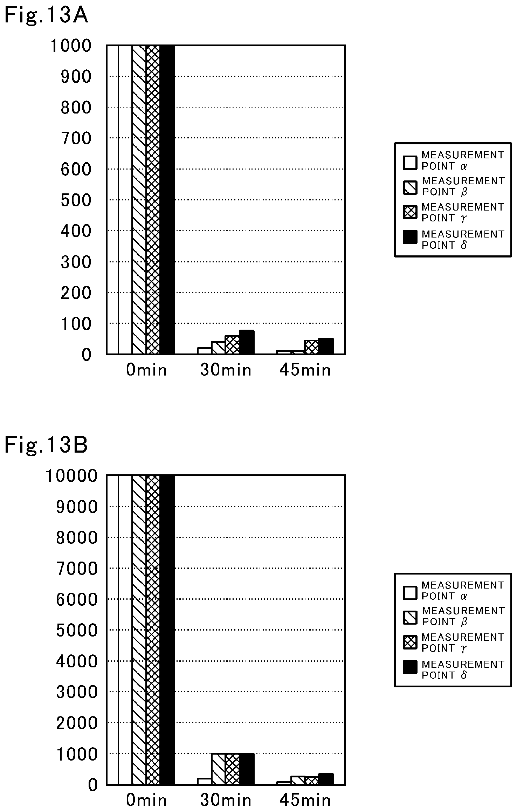

FIG. 13A is a graph for showing the results of the membrane filters to each of which a 10³ cells/ml bacterial solution was added dropwise in the case of using Example F. FIG. 13B is a graph for showing the results of the membrane filters to each of which a 10⁴ cells/ml bacterial solution was added dropwise in the case of using Example F. In FIGS. 13A and 13B, each blank portion indicates the bacterial count at the measurement point α. In FIGS. 13A and 13B, each hatching indicates the bacterial count at the measurement point β. In FIGS. 13A and 13B, each cross-hatching indicates the bacterial count at the measurement point γ. In FIGS. 13A and 13B, each solid portion indicates the bacterial count at the measurement point δ.

As shown in FIG. 13A, in Example F, when the treatment time was 0 minutes, the bacterial count was 1,058 at each of the measurement points α to δ. In addition, when the treatment time was 30 minutes, the bacterial count at the measurement point α was 20, the bacterial count at the measurement point β was 41, the bacterial count at the measurement point γ was 61, and the bacterial count at the measurement point δ was 77. When the treatment time was 45 minutes, the bacterial count at the measurement point α was 12, the bacterial count at the measurement point β was 12, the bacterial count at the measurement point γ was 48, and the bacterial count at the measurement point δ was 50.

As shown in FIG. 13B, in Example F, when the treatment time was 0 minutes, the bacterial count was 10,600 at each of the measurement points α to δ. In addition, when the treatment time was 30 minutes, the bacterial count at the measurement point α was 216, the bacterial count at the measurement point β was 1,058, the bacterial count at the measurement point γ was 1,058, and the bacterial count at the measurement point δ was 1,058. When the treatment time was 45 minutes, the bacterial count at the measurement point α was 100, the bacterial count at the measurement point β was 314, the bacterial count at the measurement point γ was 294, and the bacterial count at the measurement point δ was 355.

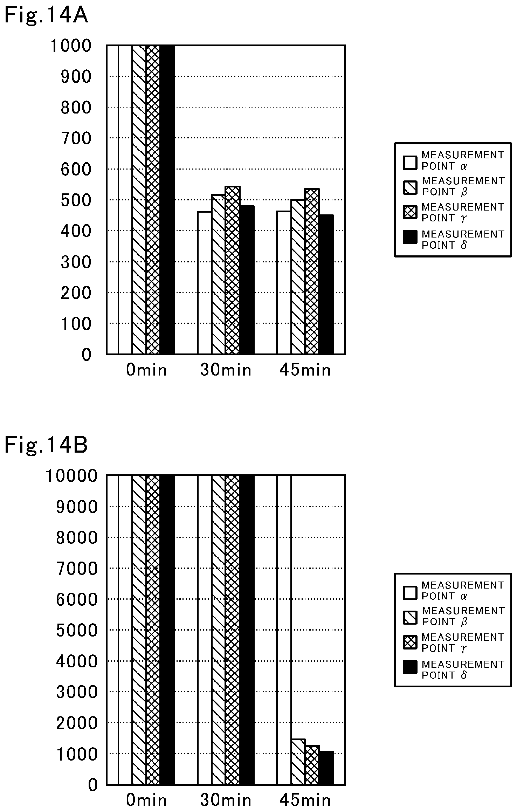

FIG. 14A is a graph for showing the results of the membrane filters to each of which a 10³ cells/ml bacterial solution was added dropwise in the case of using Comparative Example C. FIG. 14B is a graph for showing the results of the membrane filters to each of which a 10⁴ cells/ml bacterial solution was added dropwise in the case of using Comparative Example C. In FIGS. 14A and 14B, each blank portion indicates the bacterial count at the measurement point α. In FIGS. 14A and 14B, each hatching indicates the bacterial count at the measurement point β. In FIGS. 14A and 14B, each cross-hatching indicates the bacterial count at the measurement point γ. In FIGS. 14A and 14B, each solid portion indicates the bacterial count at the measurement point δ.

As shown in FIG. 14A, in Comparative Example C, when the treatment time was 0 minutes, the bacterial count was 1,235 at each of the measurement points α to δ. In addition, when the treatment time was 30 minutes, the bacterial count at the measurement point α was 458, the bacterial count at the measurement point β was 512, the bacterial count at the measurement point γ was 540, and the bacterial count at the measurement point δ was 480. When the treatment time was 45 minutes, the bacterial count at the measurement point α was 460, the bacterial count at the measurement point β was 500, the bacterial count at the measurement point γ was 532, and the bacterial count at the measurement point δ was 450.

As shown in FIG. 14B, in Comparative Example C, when the treatment time was 0 minutes, the bacterial count was 12,350 at each of the measurement points α to δ. In addition, when the treatment time was 30 minutes, the bacterial count at the measurement point α was 12,350, the bacterial count at the measurement point β was 12,350, the bacterial count at the measurement point γ was 12,350, and the bacterial count at the measurement point δ was 12,350. When the treatment time was 45 minutes, the bacterial count at the measurement point α was 12,350, the bacterial count at the measurement point β was 1,512, the bacterial count at the measurement point γ was 1,304, and the bacterial count at the measurement point δ was 1,094.

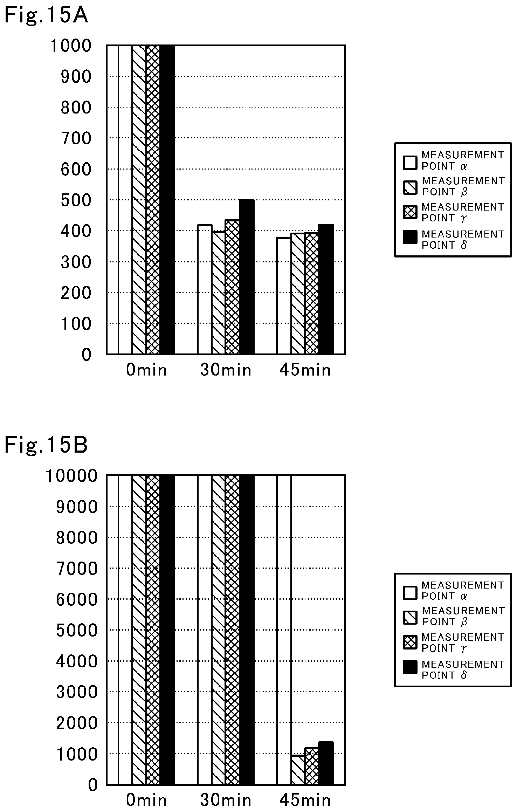

FIG. 15A is a graph for showing the results of the membrane filters to each of which a 10³ cells/ml bacterial solution was added dropwise in the case of using Comparative Example D. FIG. 15B is a graph for showing the results of the membrane filters to each of which a 10⁴ cells/ml bacterial solution was added dropwise in the case of using Comparative Example D. In FIGS. 15A and 15B, each blank portion indicates the bacterial count at the measurement point α. In FIGS. 15A and 15B, each hatching indicates the bacterial count at the measurement point β. In FIGS. 15A and 15B, each cross-hatching indicates the bacterial count at the measurement point γ. In FIGS. 15A and 15B, each solid portion indicates the bacterial count at the measurement point δ.

As shown in FIG. 15A, in Comparative Example D, when the treatment time was 0 minutes, the bacterial count was 1,235 at each of the measurement points α to δ. In addition, when the treatment time was 30 minutes, the bacterial count at the measurement point α was 420, the bacterial count at the measurement point β was 400, the bacterial count at the measurement point γ was 436, and the bacterial count at the measurement point δ was 500. When the treatment time was 45 minutes, the bacterial count at the measurement point α was 380, the bacterial count at the measurement point β was 392, the bacterial count at the measurement point γ was 396, and the bacterial count at the measurement point δ was 420.

As shown in FIG. 15B, in Comparative Example D, when the treatment time was 0 minutes, the bacterial count was 12,350 at each of the measurement points α to δ. In addition, when the treatment time was 30 minutes, the bacterial count at the measurement point α was 12,350, the bacterial count at the measurement point β was 12,350, the bacterial count at the measurement point γ was 12,350, and the bacterial count at the measurement point δ was 12,350. When the treatment time was 45 minutes, the bacterial count at the measurement point α was 12,350, the bacterial count at the measurement point β was 980, the bacterial count at the measurement point γ was 1,204, and the bacterial count at the measurement point δ was 1,394.

From the above-mentioned results, it was recognized that the ozone water of Example F had a sterilization effect higher than those of Comparative Example C and Comparative Example D.

### [Third Example]

A third Example is different from the second Example only in an ozone water spray system of the ozone water treatment apparatus 300, and the evaluation method is substantially the same except for the ozone water spray system. In the third Example, ultrasonic waves were applied to ozone water in which fine bubbles of ozone were generated in water having 1 mass% (10 g/l) of magnesium chloride dissolved therein, and the ozone water was sprayed so as to give a particle diameter of 10 µm or less.

1.0 ml of a 10⁴ cells/ml bacterial solution was added dropwise to a membrane filter. The membrane filter was accommodated in a perforated petri dish and installed at each of the measurement points α to δ of the target space 10.

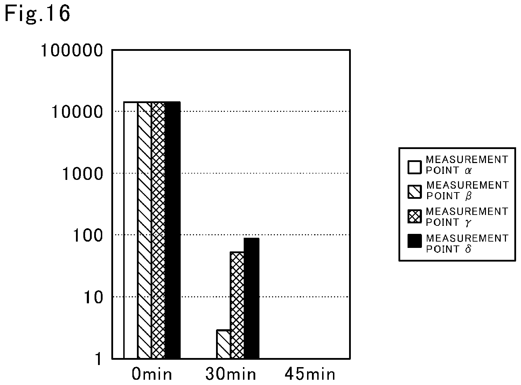

FIG. 16 is a graph for showing bacterial counts in the case of using the third Example. In FIG. 16, each blank portion indicates the bacterial count at the measurement point α. In FIG. 16, each hatching indicates the bacterial count at the measurement point β. In FIG. 16, each cross-hatching indicates the bacterial count at the measurement point γ. In FIG. 16, each solid portion indicates the bacterial count at the measurement point δ.

As shown in FIG. 16, in the third Example, when the treatment time was 0 minutes, the bacterial count was 13,000 at each of the measurement points α to δ. In addition, when the treatment time was 30 minutes, the bacterial count at the measurement point α was 0, the bacterial count at the measurement point β was 3, the bacterial count at the measurement point γ was 60, and the bacterial count at the measurement point δ was 100. When the treatment time was 45 minutes, the bacterial count at the measurement point α was 0, the bacterial count at the measurement point β was 0, the bacterial count at the measurement point γ was 0, and the bacterial count at the measurement point δ was 0.

From the above-mentioned results, it was recognized that sterilization can be performed efficiently by spraying the ozone water N so as to give a particle diameter of 10 µm or less irrespective of an atomization system. In addition, it was found that the ultrasonic system had a sterilization effect higher than that of the heating system.

The embodiment has been described above with reference to the attached drawings, but, needless to say, the present disclosure is not limited to the embodiment. It is apparent that those skilled in the art may arrive at various alternations and modifications within the scope of claims, and those examples are construed as naturally falling within the technical scope of the present disclosure.

For example, in the above-described embodiment, there is given as an example the case in which the bubble introduction unit 120 is a swirl flow type device. However, the configuration of the bubble introduction unit 120 is not limited as long as the fine bubbles of the ozone can be generated in the magnesium solution M. The bubble introduction unit 120 may be, for example, a pressurized dissolution type device.

In the above-mentioned embodiment, there is given as an example the configuration in which the ozone water treatment apparatus 300 includes the heating portion 326. However, the atomization system of the ozone water treatment apparatus is not limited as long as the ozone water N can be sprayed so as to give a particle diameter of 10 µm or less. For example, the ozone water treatment apparatus may include an ultrasonic wave applying mechanism configured to apply ultrasonic waves to the ozone water N stored in the main body 322. In this case, the spray amount of the ozone water N can be controlled by controlling the frequency (oscillation frequency) of the ultrasonic waves to be applied.

In addition, the ozone water treatment apparatus may include a two-fluid nozzle, a pressure spray valve, or a spray nozzle.

In addition, in the above-mentioned embodiment, there is given as an example the configuration in which the air blower portion 328 is provided in the main body 322. However, the air blower portion 328 may be provided outside the main body 322, for example, in the vicinity of the opening 324a of the particle diameter adjusting mechanism 324.

In addition, in the above-mentioned embodiment, there is given as an example the configuration in which the ozone water treatment apparatus 300 includes the air blower portion 328. However, the ozone water treatment apparatus 300 may not include the air blower portion 328. When the ozone water treatment apparatus 300 does not include the air blower portion 328, the particles P of the ozone water N may be diffused by air conditioning equipment that has already been installed in the target space 10.

In addition, when the ozone water treatment apparatus 300 is installed in a vehicle, air conditioning equipment of the vehicle may be operated to diffuse the particles P of the ozone water N into the air conditioning equipment as well as the inside of the vehicle. In this case, the air conditioning equipment may be set to inside-air circulation. In addition, the air conditioning equipment may be set to heating. Thus, the inside temperature rises, and the odor adsorbed to various places such as a seat and a ceiling of the vehicle is released into the inside. With this configuration, the deodorizing effect of the particles P of the ozone water N can be further improved. In addition, the effects such as sterilization and deodorization of the particles P of the ozone water N are improved along with an increase in temperature. Accordingly, the effects such as sterilization and deodorization of the particles P of the ozone water N can be further increased.

In addition, in the above-mentioned embodiment, there is given as an example the case in which the particle diameter adjusting mechanism 324 of the ozone water treatment apparatus 300 is a bellows tube. However, the particle diameter adjusting mechanism 324 may not be expandable and contractible. In this case, the length of the particle diameter adjusting mechanism 324 is set so that the particles P each having a desired particle size reach at least the opening 324a of the particle diameter adjusting mechanism 324.

In addition, in the above-mentioned embodiment, there is given as an example the configuration in which the ozone water treatment apparatus 300 includes the connection pipe 312 and the on-off valve 314. However, the mechanism for supplying the ozone water N from the ozone water tank 310 to the main body 322 is not limited. For example, an on-off valve that is opened and closed by the urging force of a spring may be provided in a lower portion of the ozone water tank 310.

In addition, there is given as an example the case in which, in the evaluation step S350 in the above-mentioned embodiment, the bacterial count of the positive control is compared to the bacterial count of each of the membrane filters installed at the measurement points α to δ. However, in the evaluation step S350, when the bacterial count counted in the counting step S340 is less than a predetermined value, it may be determined that there are effects such as sterilization and deodorization. In addition, in the evaluation step S350, when the bacterial count counted in the counting step S340 is equal to or more than the predetermined value, it may be determined that there are no effects such as sterilization and deodorization.

### Industrial Applicability

The present disclosure is applicable to an ozone water production apparatus, an ozone water production method, ozone water, an ozone water treatment apparatus, and an evaluation method.

### Reference Signs List

N: ozone water, S120: bubble introduction step, S310: installation step, S320: treatment step, S340: counting step, 100: ozone water production apparatus, 110: solution accommodation unit, 120: bubble introduction unit, 300: ozone water treatment apparatus, 320: spray unit

## Claims

1. An ozone water production apparatus, comprising:
a solution accommodation unit configured to accommodate a magnesium solution in which 1.0 mass% or more and less than 4.0 mass% of a magnesium compound is dissolved in water; and
a bubble introduction unit configured to introduce fine bubbles of ozone into the magnesium solution.

2. An ozone water production method, comprising introducing fine bubbles of ozone into a magnesium solution in which 1.0 mass% or more and less than 4.0 mass% of a magnesium compound is dissolved in water.

3. Ozone water, comprising 1.0 mass% or more and less than 4.0 mass% of a magnesium compound and fine bubbles of ozone.

4. An ozone water treatment apparatus, comprising a spray unit configured to spray ozone water containing 1.0 mass% or more and less than 4.0 mass% of a magnesium compound and fine bubbles of ozone so as to give a particle diameter of 10 µm or less.

5. An evaluation method, comprising the steps of:
installing a predetermined number of bacteria at each of a plurality of measurement points having different distances from a spray unit configured to spray ozone water containing 1.0 mass% or more and less than 4.0 mass% of a magnesium compound and fine bubbles of ozone so as to give a particle diameter of 10 µm or less;
spraying the ozone water so as to give a particle diameter of 10 µm or less by the spray unit; and
counting the number of the bacteria at each of the plurality of measurement points after an elapse of a predetermined period of time from execution of the step of spraying.
